# EUROPÄISCHE PATENTSCHRIFT

(11) **EP 2 095 787 B1**
(45) Veröffentlichungstag und Bekanntmachung des Hinweises auf die Patenterteilung: **01.09.2010**
(21) Anmeldenummer: 08152071.0
(22) Anmeldetag: 28.02.2008
(51) Int. Cl.: A61B 19/00

(54) **Verstellbare Trackingreferenz mit aushärtbarer Bonding-Verbindung**
Adjustable tracking reference with annealable bonding connection
Référence de traçage réglable dotée d'une liaison de connexion fixe pouvant être durcie

(43) Veröffentlichungstag der Anmeldung: 02.09.2009
(73) Patentinhaber: BrainLAB AG, 85622 Feldkirchen (DE)
(72) Erfinder: Plassky, Norman, 99099, Erfurt (DE); Millahn, Manuel, 80799, München (DE)
(74) Vertreter: Schwabe - Sandmair - Marx

(56) Entgegenhaltungen:
- EP-A- 0 911 668
- WO-A-91/15154
- US-A1- 2004 068 263
- US-A1- 2005 124 988
- US-A1- 2006 015 119

## Beschreibung

Die Erfindung betrifft eine verstellbare medizintechnische Trackingreferenz und ein Verfahren zum Einstellen der Ausrichtung einer solchen Trackingreferenz.

Derartige medizintechnische Trackingreferenzen werden verwendet, um mittels eines medizintechnischen Trackingsystems, das einem medizintechnischen Navigationssystem zugeordnet ist, die räumliche Lage von Objekten zu bestimmen, wobei die Trackingreferenz an einem solchen Objekt befestigt wird. Eine verstellbare Trackingreferenz mit einem Kugel Gelenk, ist aus der EP 1 563 799 A1 bekannt. Die Trackingreferenz weist einen Befestigungsarm auf, mit dem sie am Objekt befestigt wird, und einen Referenzanordnungs-Haltearm, an dem die Referenzanordnung angebracht ist oder angebracht werden kann, die wiederum vom Trackingsystem lokalisiert werden kann. Zwischen diesen beiden Armen befindet sich ein Gelenk, und dieses Gelenk dient dazu, die Referenzanordnung für verschiedene Anwendungen oder unterschiedliche Operationssaal-Setups auch unterschiedlich anordnen zu können, so dass sie in jedem Fall vom Trackingsystem erfasst werden kann.

Die Gelenke werden gemäß dem Stand der Technik beispielsweise mit Fixierschrauben ausgestattet, d. h. sie können beweglich gemacht werden, bevor der Referenzanordnungs-Haltearm richtig ausgerichtet wird. Dann müssen die Gelenke aber wieder fixiert werden und fixiert bleiben (mit der Stellschraube), damit die Navigation und das Tracking funktionieren können.

Es geschieht jedoch nicht selten, dass die behandelnden Chirurgen diese Fixierschrauben oder Einstellungsschrauben nicht oder nicht richtig fest ziehen, das Gelenk deshalb wieder lose wird und die Referenzanordnung sich während der Behandlung bewegt. Dies macht die gesamte Registrierung ungültig, und die Navigation würde zu falschen Ergebnissen führen.

Eine weitere Trackingreferenz, gemäß dem Oberbegriff des Anspruchs 1, ist aus der EP 0 911 668 bekannt.

Es ist die Aufgabe der vorliegenden Erfindung, eine medizintechnische Trackingreferenz und ein Verfahren zu ihrer Einstellung vorzuschlagen, welche ungewollte nachträgliche Verstellungen und damit verbundene Tracking- bzw. Navigationsfehler vermeidet.

Diese Aufgabe wird durch eine medizintechnische Trackingreferenz gemäß dem Anspruch 1 sowie ein Verfahren gemäß dem Anspruch 12 gelöst. Die Unteransprüche definieren bevorzugte Ausführungsformen der Erfindung.

Die erfindungsgemäße Trackingreferenz hat eine Feststellungsanordnung für das Gelenk, welche eine Bonding-Verbindung umfasst, die mit einem aushärtbarem Bondingmittel fixierbar ist. Der Begriff "Bondingmittel" umfasst hier all diejenigen Flüssigkeiten oder Feststoffe, die von einem nicht festen Zustand in einen festen Zustand übergehen und dabei Elemente in ihrer Umgebung relativ zueinander fixieren. Ob der Übergang dabei physikalisch oder chemisch angeregt bzw. unterstützt wird, ist nicht erheblich. Die Erfindung liegt also mit anderen Worten darin, dass die beiden Arme der Referenz gegeneinander fixiert werden, und zwar durch eine Verbindungstechnik, die nicht ohne Weiteres das Lösen der Verbindung gestattet, sondern die Verbindung aufrecht erhält. Damit kann das so fixierte Gelenk zwar anfänglich leicht richtig eingestellt werden; es steht aber nicht zu befürchten, dass die einmal fixierte Stellung nicht richtig befestigt wird oder wieder lösbar ist. Das ausgehärtete Bondingmittel legt die Stellung der Arme zueinander fest - versehentliche Verstellungen können nicht mehr auftreten. Das Tracking und die Navigation werden sicher und verlässlich.

Bei einer Ausführungsform der vorliegenden Erfindung ist zwischen den Armenden im bzw. am Gelenk ein Bondingraum, insbesondere ein Bondingspalt, vorhanden, der das aushärtende Bondingmittel aufnimmt und in dem die Armenden zueinander fixiert werden. Hierdurch lässt sich der Ort, wo das Bondingmittel aushärten soll, gut einstellen und kontrollieren und die ausgewählte Stellung wird zuverlässig beibehalten.

Der Bondingraum kann bei einer Erfindungsvariante mit einem Bondingmittel gefüllt sein, das durch äußere Einwirkung, insbesondere durch das Einbringen eines chemischen Stoffes oder einer Energieaufbringung von Außen, speziell in Form von Wärme- bzw. Kälteenergie, Schallenergie, elektromagnetischer Strahlungsenergie oder Lichtenergie, aushärtbar ist.

Eine erfindungsgemäße Trackingreferenz kann im oder angrenzend an den Bondingraum einen Behälter aufweisen, der das Bondingmittel umfasst und durch eine Einwirkung von Außen auf das Gelenk, insbesondere durch eine Relativbewegung der Arme, geöffnet bzw. zerstört werden kann, so dass das Bondingmittel in den Bondingraum eintritt und dort aushärtet. Ein solcher Behälter, der ein leicht zerstörbarer Glas- oder Kunststoffbehälter sein kann, kann in einer zweiten, vorzugsweise vorhandenen, Funktion noch dazu dienen, im ungeöffneten bzw. unzerstörten Zustand die Enden der Arme im Gelenk aneinander zu koppeln und relativ zueinander in einer Ausgangsstellung zu halten. Die Festigkeit des Behälters würde dann so gewählt, dass er zwar durch die Bewegung der Arme ohne Weiteres zerstört werden kann, jedoch dieser Zerstörung eine genügend große Kraft entgegenstellt, so dass die "Grundstellung" der Arme zueinander bei normalem Umgang mit der Referenz aufrechterhalten werden kann.

Das Bondingmittel kann ein Einkomponentenmittel sein, insbesondere ein Einkomponenten-Klebstoff, der nach einer vorbestimmten Zeit im Bondingraum aushärtet. Gemäß einer anderen Ausführungsform ist das Bondingmittel ein Mehrkomponentenmittel, insbesondere ein Mehrkomponenten-Klebstoff, der durch Kontakt der Komponenten nach einer vorbestimmten Zeit aushärtet, wobei insbesondere eine der Komponenten in dem vorgenannten Behälter und eine andere Komponente im Bondingraum untergebracht ist. Die Stärke der Bonding-Verbindung muss so groß sein, dass sie während der Operation eine Verstellung des Gelenkwinkels verhindert. Dies bedeutet aber nicht, dass die Aushärtung bzw. Fixierung unwiderruflich sein muss. Es ist möglich, als Bondingmittel ein replastifizierbares Mittel zu verwenden, speziell eines, das durch äußere Einwirkung, insbesondere durch das Einbringen eines chemischen Stoffes oder Energieaufbringung von Außen, beispielsweise in Form von Wärme- bzw. Kälteenergie, Schallenergie, elektromagnetische Strahlungsenergie oder Lichtenergie, wiederverflüssigbar ist. Insbesondere ist dies von Nutzen, wenn die Trackingreferenz als wiederverwendbare Referenz ausgebildet ist, wobei das Bondingmittel aus einem Material hergestellt ist, dessen Verfestigungs- und Entfestigungsfähigkeiten bei Sterilisierungstemperaturen, insbesondere über 100°C, nicht beeinträchtigt werden kann.

Bei einer anderen Ausführungsform ist die erfindungsgemäße Trackingreferenz allerdings als Wegwerf-Referenz ausgebildet, wobei insbesondere die Arme und das Gelenk aus einem Kunststoffmaterial hergestellt sind und/oder das Bondingmittel aus einem Material hergestellt ist, das bei Sterilisierungstemperaturen, insbesondere über 100°C, seine Festigkeit unwiederbringbar verliert. Eine solche Referenz kann nicht wiederverwendet werden.

Das Gelenk kann eine flexible, an den Armen endende und abdichtende Hülle aufweisen, die einen Austritt des Bondingmittels aus dem Gelenk verhindert.

Das eingangs angesprochene, erfindungsgemäße Verfahren kann durch die Verwendung einer Referenz mit den Merkmalen, wie sie oben einzeln aufgeführt wurden, gekennzeichnet sein, d. h. die Merkmale der aufgezeigten Vorrichtungen bestimmen in ihrer Eigenart auch die Ausführung der Verfahrensschritte.

Mit etwas anderen Worten lässt sich die vorliegende Erfindung auch wie folgt charakterisieren: Sie erschafft einen Mechanismus, der eine voroperative Einstellung der Referenz, jedoch keine intraoperative Bewegung mehr gestattet, und sie kann für alle Mechanismen verwendet werden, die im medizintechnischen Bereich erst eine Ersteinstellung und dann eine fixierte Position benötigen, z. B. auch bei den Gelenken für eine Einstellungsvorrichtung eines Knochenschneidblocks.

In einem ersten Zustand sind die einzustellenden Gelenke steif, sie können jedoch leicht "gebrochen" werden, beispielsweise durch das Drehen des Gelenks. Wenn das Gelenk erst einmal geöffnet ist, hat der Chirurg eine vorbestimmte Zeitspanne zur Verfügung, um den Referenzstern (Referenzanordnung) in die richtige Position zu bringen, und danach härtet das Gelenk aus und lässt keine weitere ungewollte Bewegung zu. Man kann die verschiedenen Ausführungsformen nach Wiederverwendbarkeits-Kriterien einordnen, oder beispielsweise auch nach Kriterien für das Bondingmittel (Art des Klebstoffes, Ein- oder Mehrkomponenten-Klebstoff). Ein zweikomponentiger Klebstoff würde zur Vermischung kommen, wenn das Gelenk "geöffnet", also zum ersten Mal bewegt wird, und dann aushärten. Ein einkomponentiger Klebstoff wird den Gelenkspalt nach dem "Öffnen" auffüllen, nach einer kurzen Zeit aushärten und das Gelenk so fixieren.

Wiederverwendbare Trackingreferenzen würden es gestatten, das Gelenk nach dem Aushärten wieder zu lösen und nochmals einzustellen. Der Chirurg kann die Ausrichtung des Referenzanordnungs-Haltearmes dann vorbestimmen, und zwar in Anpassung an sein persönliches OP-Setup, da dieses für ähnliche oder auch andere chirurgische Eingriffe beim selben Chirurgen ähnlich sein wird. Nachdem das Gelenk fixiert ist, hat ein Chirurg seine eigene, auf ihn abgestimmte Version, was Zeit spart und Nacheinstellungen vermeidet. In solchen Fällen wird ein Klebstoff bzw. Bondingmittel verwendet, welches Sterilisationstemperaturen widerstehen kann, und speziell bei wiederverwendbaren Referenzen hat der Chirurg auch die Möglichkeit, die Ausrichtung später neu einzustellen. Dieses Lösen des Gelenks kann, wie oben beschrieben wurde, auf unterschiedliche Arten erfolgen, und eine davon, die den Bedarf an unterschiedlichen Instrumenten im OP gering hält, ist die Verwendung eines sog. HF-Knives, welches in den meisten Operationssälen ohnehin zur Verfügung steht. Dieses HF-Knive wird als Energielieferant verwendet, um einmal gefestigte bzw. fixierte Gelenke wieder zu lösen und neu einzustellen. In der gleichen Weise kann ein Ultraschall-Abstrahler benutzt werden.

Grundsätzlich kann die Energie solcher ohnehin im OP vorhandenen Geräte auch schon vorher dazu verwendet werden, Gelenke auszuhärten. Die Gelenke könnten dann einfach mit einem Bondingmittel gefüllt sein, das beim Aufbringen von zum Beispiel HF-Strahlung oder Ultraschallwellen aushärtet.

Was die Möglichkeit angeht, die Trackingreferenz als Wegwerf-Artikel bereitzustellen, so kann die gesamte Einheit aus Kunststoff hergestellt werden, und Referenzmarker, die am Referenzanordnungs-Haltearm zu befestigen sind, werden meist ohnehin als Wegwerf-Artikel zur Verfügung gestellt. Nach dem Einsatz soll das Gelenk nicht wieder verwendet werden können, und deshalb liegt ein Sicherheitsmerkmal darin, dass der Klebstoff während des Versuchs der Dampfsterilisation zerstört und das Gelenk so gelockert wird. Es kann dann nicht mehr fixiert werden und eine zweite Verwendung wird verhindert. Die Verwendung von Kunststoffmaterialien macht die gesamte Anordnung sehr leicht, was wiederum das Risiko eines intraoperativen Lösens verringert. Außerdem kann die gesamte Referenz strahldurchgängig sein, was für spezielle Anwendungen (Fluoroskopie) von Vorteil ist.

Als Materialien für das Bondingmittel oder insbesondere den Klebstoff eignen sich Monomere, Polymere, Knochenzement, Keramik (Al₂O₃, TiO₂), Metallpulver (Ti) und Glas (SiO₂) sowie Mischungen dieser Komponenten mit Monomeren und Polymeren (Keramik, Metallpulver und Glas sind für sich alleine gesehen keine Kleber), und als Aushärtungs-, aber eventuell auch als Lösetechniken kommen im Allgemeinen Ultraschallwellen, thermische Energie, Infrarotstrahlung, Mikrowellen, UV-Strahlung und elektromagnetische Strahlung in Frage, wobei beide oben genannten Listen nicht abschließend aufzufassen sind. Hinsichtlich der Verflüssigung und Verfestigung von Materialien wird hier auf die europäische Patentanmeldung Nr. 07 122 134.5 Bezug genommen.

Die Erfindung wird im Weiteren anhand von Ausführungsbeispielen und unter Bezugnahme auf die beiliegenden Zeichnungen näher erläutert. Sie kann alle hierin beschriebenen Merkmale einzeln sowie in jedweder sinnvollen Kombination umfassen. In den beiliegenden Zeichnungen zeigen:
- Figur 1: eine medizintechnische Trackingreferenz gemäß der vorliegenden Erfindung;
- Figur 2: einen Schnitt durch ein Gelenk gemäß einer ersten Ausführungsform der Erfindung;
- Figur 3: einen Schnitt durch ein Gelenk gemäß einer zweiten Ausführungsform der Erfindung; und
- Figur 4: eine schematische Darstellung der Energieaufbringung am Referenz-Gelenk zur Phasenänderung (lose bzw. flüssig zu fest) des Bondingmittels.

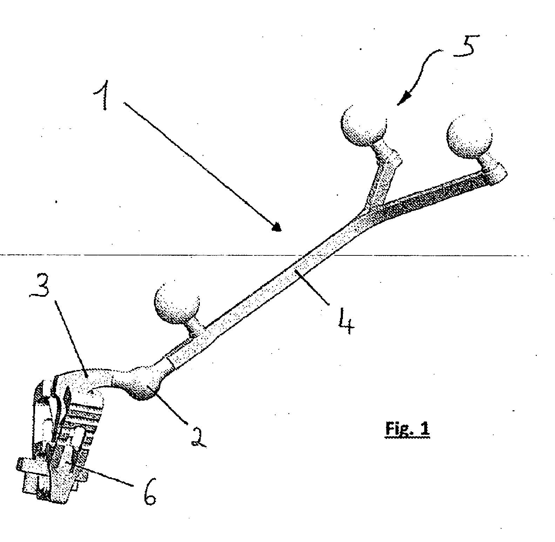

Eine medizintechnische Trackingreferenz ist in der Figur 1 gezeigt und mit dem Bezugszeichen 1 versehen. Sie weist ein Gelenk 2 auf, das zwischen einem Befestigungsarm 3 und einem Referenzanordnungs-Haltearm 4 liegt. Der Befestigungsarm 3 heißt so, weil an ihm die Befestigung 6 angebracht ist, mit welcher die Trackingreferenz 1 an einem Objekt angebracht werden kann, also beispielsweise an einem Knochen, um den Knochen tracken zu können. Der Referenzanordnungs-Haltearm 4 heißt so, weil an ihm die Referenzanordnung angebracht werden kann, die hier aus drei reflektiven Kugelmarkern besteht, von denen einer mit dem Bezugszeichen 5 versehen worden ist.

Durch die Bewegung im Gelenk 2 lässt sich der Haltearm 4 gegenüber dem Befestigungsarm 3 bewegen, und so kann die Winkeleinstellung für den Haltearm 4 optimal gewählt werden, nämlich so, dass die Reflektorenmarker 5 ohne Weiteres von einem Trackingsystem erfasst werden können (freie "Line of Sight").

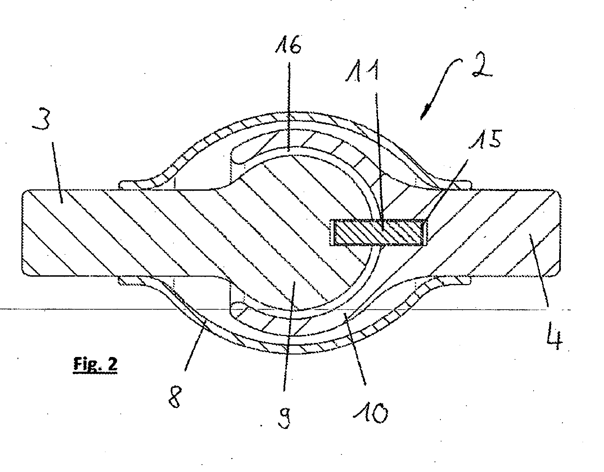

Eine Ausgestaltung eines Gelenks ist im Schnitt in Figur 2 gezeigt, wobei das Gelenk 2 als Kugelgelenk ausgebildet ist. Das Gelenk 2 umfasst das Kugelende 9 des Befestigungsarms 3 und das Schalenende 10 des Referenzanordnungs-Haltearms 4. Über dem Kugelgelenk ist eine Umhüllung 8 angebracht, die dichtend an den Armen 3, 4 abschließt und aus einem flexiblen Material gebildet ist. Zwischen der Schale 10 und der Kugel 9 ist etwas Spiel, d. h. dort bildet sich ein Gelenkspalt 16, der auch als Bondingspalt 16 bezeichnet werden kann. Zwischen dem Stirnende der Kugel 9 und dem tiefsten Punkt der Schale 10 ist jeweils in Aussparungen dieser beiden Elemente ein Behälter 15 angeordnet, der eine zerbrechliche Hülle (Glas, Kunststoff) aufweist und in dem das Bondingmittel 11, z. B. ein Einkomponenten-Klebstoff untergebracht ist.

In dem in Figur 2 dargestellten Zustand, dem Ausgangszustand, kann der Behälter 15 den Gelenken in dieser Lage eine gewisse Stabilität geben; d. h. die Ausgangslage wird erhalten, solange nicht mit größerer Kraft an der Gelenkstellung gearbeitet wird. Wird das Gelenk aber tatsächlich willentlich und mit etwas größerer Kraft in eine bestimmte von der Ausgangsstellung abweichende Stellung gebracht, wird der Behälter 15 zerstört und das Bondingmittel (Klebstoff) 11 kann in den Gelenkspalt 16 eintreten. Es wird dort nach kurzer Zeit aushärten und so die gewünschte Stellung des Gelenks fixieren. Diese Stellung kann dann nicht mehr durch versehentliche Berührungen der Referenz 1 verändert werden, und es besteht auch nicht die Gefahr, dass das Fixieren der Gelenkstellung vergessen wird, weil es einfach durch die Aushärtung des Bondingmittels selbst passiert.

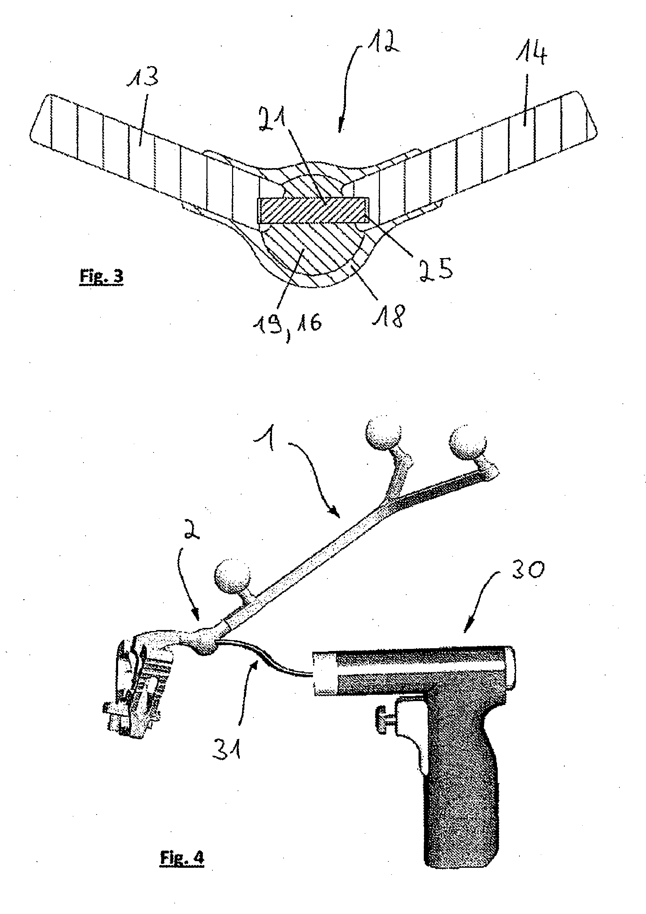

Eine weitere Ausführungsform eines verwendbaren Gelenks ist im Schnitt in der Figur 3 gezeigt; das Gelenk hat das Bezugszeichen 12 und die beiden Arme sind mit 13 und 14 benannt. Das Gelenk ist wiederum mit einer Umhüllung 18 umgeben, die an den Armen 13, 14 abdichtet, und innerhalb der Umhüllung 18 befindet sich eine Komponente 19 eines Bondingmittels oder Klebstoffs im Bondingraum 16. Zwischen den Stirnenden der Arme 13, 14 und wiederum in Aussparungen dort, ist der Behälter 25 gelagert, der wiederum die zweite Komponente 21 des Zweikomponenten-Bondingmittels 19, 21 aufweist. Wie bei der Ausführungsform nach Figur 2 wird der Behälter 25 dann zerstört, wenn die beiden Arme 13, 14 bewegt, zum Beispiel in ihre Endposition gestellt werden, die beiden Komponenten 19, 21 des Bondingmittels vermischen sich und härten aus, und sie halten dann die Stirnenden der Arme 13, 14 im Gelenk fest, so dass diese Endstellung nicht mehr verändert wird.

In der Figur 4 ist die Trackingreferenz 1 und ein Ultraschall-Emissionsgerät 30 gezeigt, und mit der Verbindung 31 soll angedeutet werden, dass dieses Ultraschallgerät seine Wellen auf das Gelenk hin ausstrahlen kann. Damit können die beiden folgenden Situationen verdeutlicht werden:
1. Aushärtung durch Ultraschallwellen: Es ist möglich, ein Bondingmittel, das sich im Gelenk im Bondingraum befindet, durch die Energie des Ultraschallstrahlers 30 auszuhärten, wenn die Arme vorher in die gewünschte Position gebracht worden sind. Dies wäre also eine Ausgestaltungsform der Erfindung, bei der ein (zum Beispiel einkomponentiges) ultraschallhärtendes Bondingmittel im Bondingraum des Gelenks 2 verwendet wird und in einfacher Weise eine Aushärtung erfolgen kann.
2. Entfestigung (Lösen) des Gelenks: In umgekehrter Weise könnte die Figur 4 auch darstellen, dass mittels einer Ultraschallabstrahlung ein vorher schon einmal eingestelltes Gelenk wieder entfestigt wird, d. h. beispielsweise wird ein verfestigtes Bondingmittel im Bondingraum wieder verflüssigt, so dass eine neue Einstellung wählbar ist. Ein ganz einfaches Beispiel wäre hier auch eine Masse, die durch Wärme plastifizierbar ist und als Bondingmittel verwendet wird. Im kalten Zustand würde das Gelenk fest eingestellt sein, durch Wärmeeinwirkung (z.B. Mikrowellen) würde es gelöst, und wenn man es danach wieder abkühlen lässt, würde das Gelenk wieder fixiert.

## Patentansprüche

1. Medizintechnische Trackingreferenz (1) mit einem Befestigungsarm (3) und einem Referenzanordnungs-Haltearm (4), an dem eine Referenzanordnung (5) angebracht ist oder angebracht werden kann, und mit einem Gelenk (2) zwischen den Armen (3, 4), und mit einer Feststellanordnung mit der das Gelenk fixiert werden kann, **dadurch gekennzeichnet, dass** das Gelenk (2) ein Kugelgelenk ist und die Feststellungsanordnung des Kugelgelenks (2) eine Bonding-Verbindung mit einem aushärtbaren Bondingmittel (11; 21, 19) umfasst und mit dem Bondingmittel fixierbar ist.

2. Medizintechnische Trackingreferenz (1) nach Anspruch 1, **dadurch gekennzeichnet, dass** zwischen den Armenden (9, 10) im bzw. am Gelenk (2) ein Bondingraum (16), insbesondere ein Bondingspalt, vorhanden ist, der das aushärtende Bondingmittel (11; 21; 19) aufnimmt und in dem die Armenden (9, 10) zueinander fixiert werden.

3. Medizintechnische Trackingreferenz (1) nach Anspruch 2, **dadurch gekennzeichnet, dass** der Bondingraum mit einem Bondingmittel gefüllt ist, das durch äußere Einwirkung, insbesondere durch das Einbringen eines chemischen Stoffes oder Energieaufbringung von Außen, speziell in Form von Wärme- bzw. Kälteenergie, Schallenergie, elektromagnetischer Strahlungsenergie oder Lichtenergie, aushärtbar ist.

4. Medizintechnische Trackingreferenz (1) nach einem der Ansprüche 2 bis 3, **dadurch gekennzeichnet, dass** im oder angrenzend an den Bondingraum (16) ein Behälter (15; 25) angeordnet ist, der das Bondingmittel umfasst und durch eine Einwirkung von Außen auf das Gelenk, insbesondere durch eine Relativbewegung der Arme (3, 4), geöffnet bzw. zerstört werden kann, so dass das Bondingmittel in den Bondingraum (16) eintritt und dort aushärtet.

5. Medizintechnische Trackingreferenz (1) nach Anspruch 4, **dadurch gekennzeichnet, dass** der Bondingmittel-Behälter im ungeöffneten bzw. unzerstörten Zustand die Enden der Arme (3, 4) im Gelenk aneinander koppelt und relativ zueinander in einer Ausgangsstellung hält.

6. Medizintechnische Trackingreferenz (1) nach Anspruch 4 oder 5, **dadurch gekennzeichnet, dass** das Bondingmittel ein Einkomponentenmittel (11), insbesondere ein Einkomponenten-Klebstoff ist, der nach einer vorbestimmten Zeit im Bondingraum (15) aushärtet.

7. Medizintechnische Trackingreferenz (1) nach Anspruch 4 oder 5, **dadurch gekennzeichnet, dass** das Bondingmittel ein Mehrkomponentenmittel (21, 19), insbesondere ein Mehrkomponenten-Klebstoff, ist, der durch Kontakt der Komponenten nach einer vorbestimmten Zeit aushärtet, wobei insbesondere eine der Komponenten (21) in dem Behälter (15) und eine andere Komponente im Bondingraum (19) untergebracht ist.

8. Medizintechnische Trackingreferenz (1) nach einem der Ansprüche 1 bis 7, **dadurch gekennzeichnet, dass** das Bondingmittel ein replastifizierbares Mittel ist, speziell eines, das durch äußere Einwirkung, insbesondere durch das Einbringen eines chemischen Stoffes oder Energieaufbringung von Außen, beispielsweise in Form von Wärme- bzw. Kälteenergie, Schallenergie, elektromagnetischer Strahlungsenergie oder Lichtenergie, wiederverflüssigbar ist.

9. Medizintechnische Trackingreferenz (1) nach einem der Ansprüche 1 bis 8, **dadurch gekennzeichnet, dass** sie als wiederverwendbare Referenz ausgebildet ist, wobei das Bondingmittel aus einem Material hergestellt ist, dessen Verfestigungs- und Entfestigungsfähigkeiten bei Sterilisierungstemperaturen, insbesondere über 100°C, nicht beeinträchtigt werden.

10. Medizintechnische Trackingreferenz (1) nach einem der Ansprüche 1 bis 7, **dadurch gekennzeichnet, dass** sie als Wegwerf-Referenz ausgebildet ist, wobei insbesondere die Arme (3, 4; 13, 14) und das Gelenk aus einem Kunststoffmaterial hergestellt sind und/oder das Bondingmittel aus einem Material hergestellt ist, das bei Sterilisierungstemperaturen, insbesondere über 100°C, seine Festigkeit unwiederbringbar verliert.

11. Medizintechnische Trackingreferenz (1) nach einem der Ansprüche 1 bis 10, **dadurch gekennzeichnet, dass** das Gelenk (2; 12) eine flexible, an den Armen (3, 4; 13, 14) endende und abdichtende Hülle (8; 18) aufweist.

12. Verfahren zum voroperativen Einstellen der Ausrichtung einer medizintechnischen Trackingreferenz (1) mit einem Befestigungsarm (3) und einem Referenzanordnungs-Haltearm (4), an dem eine Referenzanordnung (5) angebracht ist oder angebracht werden kann, und mit einem Gelenk (2) zwischen den Armen (3, 4), und mit einer Feststellanordnung mit der das Gelenk fixiert werden kann, **dadurch gekennzeichnet, dass** das Gelenk (2) ein Kugelgelenk ist und die Feststellungsanordnung des Kugelgelenks (2) ein aushärtbares Bondingmittel (11; 21, 19) umfasst und **dadurch** fixiert wird, wobei das Bondingmittel eine Bonding-Verbindung aufbaut.

## Claims

1. A medical tracking reference (1), comprising a fastening arm (3) and a reference array holding arm (4) to which a reference array (5) is or can be attached, and comprising a joint (2) between the arms (3, 4), and comprising a fixing array using which the joint can be fixed, **characterised in that** the joint (2) is a ball joint and the fixing array of the ball joint (2) comprises a bonding connection comprising a curable bonding agent (11; 21, 19) and can be fixed using the bonding agent.

2. The medical tracking reference (1) according to claim 1, **characterised in that** a bonding space (16) - in particular, a bonding gap - is provided in and/or on the joint (2), between the ends (9, 10) of the arms, which accommodates the curing bonding agent (11; 21, 19) and in which the ends (9, 10) of the arms are fixed relative to each other.

3. The medical tracking reference (1) according to claim 2, **characterised in that** the bonding space is filled with a bonding agent which can be cured by an external influence, in particular by introducing a chemical substance or by applying energy from without, specifically in the form of heating and/or cooling energy, sonic energy, electromagnetic radiation energy or light energy.

4. The medical tracking reference (1) according to any one of claims 2 to 3, **characterised in that** a container (15; 25) is arranged in or adjacent to the bonding space (16) and comprises the bonding agent and can be opened and/or destroyed by an influence on the joint from without, in particular by a relative movement of the arms (3, 4), such that the bonding agent enters the bonding space (16), where it cures.

5. The medical tracking reference (1) according to claim 4, **characterised in that** in its unopened and/or non-destroyed state, the bonding agent container couples the ends of the arms (3, 4) to each other in the joint and holds them relative to each other in an initial placement.

6. The medical tracking reference (1) according to claim 4 or 5, **characterised in that** the bonding agent is a one-component agent (11), in particular a one-component glue, which cures in the bonding space (15) after a predetermined period of time.

7. The medical tracking reference (1) according to claim 4 or 5, **characterised in that** the bonding agent is a multiple-component agent (21, 19), in particular a multiple-component glue, which cures after a predetermined period of time due to contact between the components, wherein in particular one of the components (21) is accommodated in the container (15) and another component is accommodated in the bonding space (19).

8. The medical tracking reference (1) according to any one of claims 1 to 7, **characterised in that** the bonding agent is a replastifiable agent, specifically one which can be re-liquefied by an external influence, in particular by introducing a chemical substance or by applying energy from without, for example in the form of heating and/or cooling energy, sonic energy, electromagnetic radiation energy or light energy.

9. The medical tracking reference (1) according to any one of claims 1 to 8, **characterised in that** it is designed as a reusable reference, wherein the bonding agent is made from a material whose hardening and softening capabilities are not impaired at sterilisation temperatures, in particular above 100°C.

10. The medical tracking reference (1) according to any one of claims 1 to 7, **characterised in that** it is designed as a disposable reference, wherein the arms (3, 4; 13, 14) and the joint are in particular made from a plastic material and/or the bonding agent is made from a material which irretrievably loses its strength and/or solidity at sterilisation temperatures, in particular above 100°C.

11. The medical tracking reference (1) according to any one of claims 1 to 10, **characterised in that** the joint (2; 12) comprises a flexible sleeve (8; 18) which ends at the arms (3, 4; 13, 14) and seals them.

12. A method for pre-operatively setting the alignment of a medical tracking reference (1) comprising a fastening arm (3) and a reference array holding arm (4) to which a reference array (5) is or can be attached, and comprising a joint (2) between the arms (3, 4), and comprising a fixing array using which the joint can be fixed, **characterised in that** the joint (2) is a ball joint and the fixing array of the ball joint (2) comprises a curable bonding agent (11; 21, 19) by which it is fixed, wherein the bonding agent establishes a bonding connection.

## Revendications

1. Référence de repérage médico-technique (1) comportant un bras de fixation (3) et un bras de support d'agencement de référence (4) sur lequel un agencement de référence (5) est fixé ou peut être fixé, et comportant une articulation (2) entre les bras (3, 4), et comportant un agencement de fixation avec lequel l'articulation peut être fixée, **caractérisée en ce que** l'articulation (2) est une articulation sphérique et l'agencement de fixation de l'articulation sphérique (2) comporte une liaison adhésive avec un adhésif durcissable (11 ; 21, 19) et peut être fixée à l'aide de l'adhésif.

2. Référence de repérage médico-technique (1) selon la revendication 1, **caractérisée en ce qu'**il existe un espace d'adhérence (16) entre les extrémités de bras (9, 10) dans ou sur l'articulation (2), en particulier un intervalle d'adhérence, qui reçoit l'adhésif durcissant (11 ; 21, 19) et dans lequel les extrémités de bras (9, 10) sont fixées l'une à l'autre.

3. Référence de repérage médico-technique (1) selon la revendication 2, **caractérisée en ce que** l'espace d'adhérence est rempli d'un adhésif qui peut durcir par une influence extérieure, en particulier par l'introduction d'une substance chimique ou un apport d'énergie provenant de l'extérieur, notamment sous forme d'énergie de chauffage ou de refroidissement, d'énergie acoustique, d'énergie de rayonnement électromagnétique ou d'énergie lumineuse.

4. Référence de repérage médico-technique (1) selon l'une quelconque des revendications 2 à 3, **caractérisée en ce qu'**un conteneur (15 ; 25) est disposé dans l'espace d'adhérence (16) ou adjacent à celui-ci, ledit conteneur contenant l'adhésif et pouvant être ouvert ou détruit par une influence exercée sur l'articulation et provenant de l'extérieur, en particulier par un mouvement relatif des bras (3, 4), de telle sorte que l'adhésif pénètre dans l'espace d'adhérence (16) et y durcit.

5. Référence de repérage médico-technique (1) selon la revendication 4, **caractérisée en ce que** le conteneur d'adhésif à l'état non ouvert ou non détruit couple les extrémités des bras (3, 4) l'une à l'autre dans l'articulation et les maintient l'une par rapport à l'autre dans une position initiale.

6. Référence de repérage médico-technique (1) selon la revendication 4 ou 5, **caractérisée en ce que** l'adhésif est un agent à un seul composant (11), en particulier une colle à un seul composant, qui durcit après un temps prédéterminé dans l'espace d'adhérence (15).

7. Référence de repérage médico-technique (1) selon la revendication 4 ou 5, **caractérisée en ce que** l'adhésif est un agent à plusieurs composants (21, 19), en particulier une colle à plusieurs composants, qui durcit après un temps prédéterminé par mise en contact des composants, dans laquelle en particulier l'un des composants (21) est reçu dans le conteneur (15) et un autre composant est reçu dans l'espace d'adhérence (19).

8. Référence de repérage médico-technique (1) selon l'une quelconque des revendications 1 à 7, **caractérisée en ce que** l'adhésif est un agent replastifiable, notamment un agent qui peut être re-liquéfié par une influence extérieure, en particulier par l'introduction d'une substance chimique ou un apport d'énergie provenant de l'extérieur, par exemple sous forme d'énergie de chauffage ou de refroidissement, d'énergie acoustique, d'énergie de rayonnement électromagnétique ou d'énergie lumineuse.

9. Référence de repérage médico-technique (1) selon l'une quelconque des revendications 1 à 8, **caractérisée en ce qu'**elle est conçue sous forme de référence réutilisable, dans laquelle l'adhésif est fabriqué à partir d'un matériau dont les capacités de durcissement et d'amollissement ne sont pas dégradées à des températures de stérilisation, en particulier des températures supérieures à 100 °C.

10. Référence de repérage médico-technique (1) selon l'une quelconque des revendications 1 à 7, **caractérisée en ce qu'**elle est conçue sous forme de référence jetable, dans laquelle en particulier les bras (3, 4 ; 13, 14) et l'articulation sont fabriqués à partir d'une matière plastique et/ou l'adhésif est fabriqué à partir d'un matériau qui perd irrémédiablement sa résistance à des températures de stérilisation, en particulier des températures supérieures à 100 °C.

11. Référence de repérage médico-technique (1) selon l'une quelconque des revendications 1 à 10, **caractérisée en ce que** l'articulation (2 ; 12) comporte un manchon souple (8 ; 18) qui étanchéifie les bras (3, 4 ; 13, 14) et se termine à leur niveau.

12. Procédé pour régler de manière préopératoire l'alignement d'une référence de repérage médico-technique (1) comportant un bras de fixation (3) et un bras de support d'agencement de référence (4) sur lequel un agencement de référence (5) est monté ou peut être monté, et comportant une articulation (2) entre les bras (3, 4), et comportant un agencement de fixation avec lequel l'articulation peut être fixée, **caractérisé en ce que** l'articulation (2) est une articulation sphérique et l'agencement de fixation de l'articulation sphérique (2) comporte un adhésif durcissable (11; 21, 19) et est ainsi fixé, l'adhésif établissant une liaison adhésive.
